# EUROPÄISCHE PATENTANMELDUNG

(11) **EP 3 772 361 A1**
(43) Veröffentlichungstag der Anmeldung: **10.02.2021**
(21) Anmeldenummer: 19190314.5
(22) Anmeldetag: 06.08.2019
(51) Int. Cl.: A61N 1/39, H03K 3/015, H03K 3/57, H02M 3/07, H02J 7/00, H02J 7/34

(54) **IMPLANTIERBARER IMPULSGENERATOR MIT RECHTECKIGER SCHOCKFORM**

(71) Anmelder: BIOTRONIK SE & Co. KG, 12359 Berlin (DE)
(72) Erfinder: Dörr, Thomas, 12437 Berlin (DE); Weiss, Ingo, 12435 Berlin (DE)
(74) Vertreter: Galander, Marcus

(57) **Zusammenfassung**

Die vorliegende Erfindung betrifft einen implantierbaren Impulsgenerator mit einer elektrischen Schaltung, wobei die elektrisch Schaltung umfasst: einen primären Energiespeicher, mindestens einen sekundäre Energiespeicher, eine Steuereinheit, wobei die Steuereinheit dazu ausgebildet ist, einen elektrischen Schalter in der elektrischen Schaltung derart anzusteuern, dass in einem ersten Intervall einer ersten Phase einer Impulsabgabe der primäre Energiespeicher über einen therapeutischen Strompfad entladen wird, und einen elektrischen Schalter in der elektrischen Schaltung derart anzusteuern, dass in einem zweiten Intervall der ersten Phase der Impulsabgabe der sekundäre Energiespeicher über den therapeutischen Strompfad entladen wird,

## Beschreibung

Die vorliegende Erfindung betrifft einen implantierbaren Impulsgenerator.

Bislang werden in implantierbaren Defibrillatoren (ICDs) immer Kondensatorentladungen zur Defibrillation genutzt, wobei die gesamte Schockenergie aus einer konstanten Kapazität über den Verlauf der Defibrillation entnommen wird. Charakteristisch dafür ist der exponentiell abfallende Spannungsverlauf.

Bei externen Defibrillatoren werden bereits Schockformen eingesetzt, die eine minimierte Schockspannung unterstützen.

Nachteil der exponentiellen Schockform sind die benötigten hohen Spitzenspannungen, um eine effektive Defibrillation durchzuführen. Die führt insbesondere bei nicht-transvenösen Defibrillatoren (z.B. subkutane ICDs wie S-ICD™) zu Spitzenspannungen von über 1300 V, welche wiederum entsprechende Hochspannungskomponenten und die Einhaltung angepasster Designregeln erfordern.

Basierend darauf ist eine Aufgabe der vorliegenden Erfindung einen implantierbaren Pulsgenerator zur Verfügung zu stellen, welcher mit geringerer Schockspannung einen therapeutisch wirksamen Schock zur Verfügung stellen kann.

Diese Aufgabe wird durch einen implantierbaren Impulsgenerator mit den Merkmalen des Anspruchs 1 und ein Verfahren mit den Merkmalen des Anspruchs 11 gelöst. Geeignete Ausführungsformen sind in den abhängigen Ansprüchen und der folgenden Beschreibung angegeben.

Gemäß Anspruch 1 wird ein implantierbaren Impulsgenerator mit einer elektrischen Schaltung zur Verfügung gestellt. Die elektrische Schaltung umfasst:
- einen primären Energiespeicher,
- mindestens einen sekundären Energiespeicher, und
- eine Steuereinheit, wobei die Steuereinheit, dazu ausgebildet ist
   - einen elektrischen Schalter in der elektrischen Schaltung derart anzusteuern, dass in einem ersten Intervall einer ersten Phase einer Impulsabgabe der primäre Energiespeicher über einen therapeutischen Strompfad entladen wird, und
   - einen elektrischen Schalter in der elektrischen Schaltung derart anzusteuern, dass in einem zweiten Intervall der ersten Phase der Impulsabgabe der mindestens eine sekundäre Energiespeicher über den therapeutischen Strompfad entladen wird.

Insbesondere dient der therapeutische Strompfad zur Abgabe eines therapeutischen elektrischen Impulses in ein Zielgewebe, vorzugsweise das Herzgewebe eines Patienten, vorzugsweise zur Abgabe eines therapeutischen elektrischen Schocks zur Defibrillation des Herzgewebes.

Weiterhin werden insbesondere der primäre Energiespeicher und der mindestens eine sekundäre Energiespeicher im zweiten Intervall der ersten Phase der Impulsabgabe über den therapeutischen Strompfad entladen.

Vorteilhafterweise kann mit dem erfindungsgemäßen Impulsgenerator eine Schockabgabe mit annährend rechteckigem Impulsverlauf erreicht werden, insbesondere eines im Wesentlichen rechteckigen Spannungs- oder Stromverlaufs während der Impulsangabe. Damit ist über eine definierte Zeit die Abgabe eines therapeutisch wirksamen (defibrillierenden) Impulses bei Reduzierung der maximalen benötigen Schockspannung möglich. Vorteilhafterweise gestattet die erfindungsgemäße Lösung insbesondere durch Reduzierung der maximal benötigten Schockspannung einen kostengünstigeren Aufbau für implantierbare Defibrillatoren und die Nutzung bereits existierender Hochspannungskomponenten und Plattformen.

Der erfindungsgemäße implantierbare Impulsgenerator eignet sich daher insbesondere zur Verwendung als Kardioverter-Defibrillator, dass insbesondere mit der erfindungsgemäßen elektrischen Schaltungen Therapiespannungen von bis zu 1200°V mit dem oben beschriebenen rechteckigen Impulsverlauf erzielt werden können. Damit können der erfindungsgemäße Impulsgenerator als ein Elektrodenpol und das Ende einer mit dem Impulsgenerator verbundenen Elektrodenleitung als der andere Elektrodenpol außerhalb des Brustkorbes eines Patienten angeordnet werden (subkutaner ICD). Vorteilhafterweise weist ein als subkutaner ICD ausgestalteter Impulsgenerator aufgrund der möglichen kompakteren Bauweise ein Volumen von 70 cm³ oder weniger auf.

Natürlich können mit dem erfindungsgemäßen Impulsgenerator auch geringere Therapiespannung erreicht werden, beispielsweise 600 V, was eine Verwendung als konventioneller ICD ermöglicht, wobei das Ende der Elektrodenleitung innerhalb des Brustkorbs angeordnet ist, und der Impulsgenerator außerhalb des Brustkorbs liegt. Die vorgenannte geringere Therapiespannung kann durch kleinere Energiespeicher realisiert werden. Dementsprechend kann ein als konventioneller ICD ausgestalteter Impulsgenerator aufgrund der möglichen kompakteren Bauweise ein Volumen von 35 cm³ oder weniger aufweisen.

Auch kann der erfindungsgemäße Impulsgenerator in einem ICD verwendet werden, bei dem sowohl das Ende der Elektrodenleitung als auch der Impulsgenerator innerhalb des Brustkorbs liegen. In diesem Fall wird vorzugsweise eine Therapiespannung von etwa 550 V verwendet, welches mit den erfindungsgemäßen Impulsgenerator mit kleineren Energiespeichern realisiert werden, so dass insbesondere ein Gerätevolumen von 35 cm² oder weniger erreicht werden kann.

Gemäß einer Ausfuhrungsform des erfindungsgemäßen implantierbaren Impulsgenerators ist vorgesehen, dass der primäre Energiespeicher und der mindestens eine sekundäre Energiespeicher in Reihe geschaltet sind, insbesondere fest oder unveränderlich in Reihe, d. h. die vorgenannten Energiespeicher sind nicht durch einen Schalter getrennt. Durch diese feste Reihenschaltung ist die erfindungsgemäße elektrische Schaltung vorteilhafterweise weniger fehleranfällig.

Gemäß einer alternativen Ausführungsform des implantierbaren Impulsgenerators ist vorgesehen, dass der primäre Energiespeicher und der mindestens eine sekundäre Energiespeicher in Reihe schaltbar sind.

Gemäß einer Ausfuhrungsform des erfindungsgemäßen implantierbaren Impulsgenerators ist vorgesehen, dass das Ansteuern zur Entladung des mindestens einen sekundären Energiespeichers zeitgesteuert oder signalgesteuert erfolgt.

Hierbei kann sich das Steuersignal aus einer Analyse einer oder mehreren Messspannungen oder -ströme ableiten, die an den Energiespeichern selbst oder an dem abgegebenen Impuls, beispielsweise im therapeutischen Strompfad, gemessen werden.

Dementsprechend umfasst gemäß einer Ausführungsform der erfindungsgemäßen implantierbaren Impulsgenerators eine Vorrichtung zur Spannungsüberwachung, die insbesondere dazu ausgebildet ist, die Spannung jedes der Energiespeicher des erfindungsgemäßen implantierbaren Impulsgenerators zu bestimmen, vorzugsweise kontinuierlich oder in vordefinierten Intervallen.

Vorzugsweise ist die Vorrichtung zur Spannungsüberwachung weiterhin dazu ausgebildet, ein erstes Signal an die Steuereinheit zu übermitteln, wenn die Spannung eines der Energiespeicher des erfindungsgemäßen implantierbaren Impulsgenerators einen vordefinierten Schwellenwert unterschreitet. Vorteilhafterweise kann das erste Signal dabei die Entladung eines weiteren Energiespeichers über den therapeutischen Strompfades auslösen.

Vorzugsweise ist die Vorrichtung zur Spannungsüberwachung auch dazu ausgebildet, ein zweites Signal an die Steuereinheit zu übermitteln, wenn die Spannung eines der Energiespeicher des erfindungsgemäßen implantierbaren Impulsgenerators innerhalb einer vordefinierten Zeit nicht unterhalb eines vordefinierten Schwellenwertes sinkt. Vorteilhafter kann das zweite Signal eine Entladung eines weiteren Energiespeichers über den therapeutischen Strompfad unterbinden. In dieser Ausgestaltung können die Vorrichtung zur Spannungsüberwachung und die Steuereinheit einen Überspannungsschutz insbesondere für die erfindungsgemäße elektrische Schaltung realisierten. Dabei wird über die Spannungsmessung festgestellt, ob eine Last auf dem therapeutischen Stompfad liegt (Spannungsabfall) oder keine Last (kein Spannungsabfall). Falls keine Last festgestellt wird, werden keine weiteren Energiespeicher mehr über den therapeutischen Strompfad entladen, um die Spannung, beispielsweise im therapeutischen Strompfad, nicht weiter zu erhöhen.

Gemäß eine weiteren Ausführungsform umfasst der erfindungsgemäße implantierbare Impulsgenerator eine Vorrichtung zur Impedanzmessung des umliegenden Gewebes (Körpergewebe). Vorzugsweise ist die Vorrichtung zur Impedanzmessung dazu ausgebildet, aus einer nach Beginn der Entladung des primären Energiespeichers und/oder des mindestens einen sekundären Energiespeicher ermittelten Spannung den Widerstand zu bestimmen, der die Entladung (beobachtet durch den Spannungsabfall) verursacht. Die Spannung kann dabei die Therapiespannung, die Spannung eines individuellen Energiespeichers oder eine irgendeine Spannung innerhalb der erfindungsgemäßen elektrischen Schaltung sein. Die Spannung kann dabei durch die vorgenannte Vorrichtung zur Spannungsüberwachung ermittelt werden.

Gemäß einer weiteren Ausführungsform des erfindungsgemäßen implantierbaren Impulsgenerators ist vorgesehen, dass der Zeitpunkt für den Beginn des zweiten Intervalls der ersten Phase aus dem Entladeverhalten des ersten Energiespeichers und/oder des mindestens einen sekundären Energiespeicher bestimmt wird. Hierbei wird der vorgenannte Zeitpunkt derartig gewählt, dass die Spannung des betreffenden Energiespeichers nicht unter einen vordefinierten Schwellenwert fällt, beispielsweise 80 % der Ausgangsspannung.

Gemäß einer weiteren Ausführungsform des erfindungsgemäßen implantierbaren Impulsgenerators ist vorgesehen, dass die elektrische Schaltung mehrere sekundäre Energiespeicher aufweist, wobei die Steuereinheit dazu ausgebildet ist, einen oder mehrere elektrische Schalter der elektrischen Schaltung derart anzusteuern, dass im zweiten Intervall der ersten Phase der Pulsabgabe die sekundären Energiespeicher sequentiell oder hintereinander über den therapeutischen Strompfad entladen werden.

Gemäß einer weiteren Ausführungsform des erfindungsgemäßen implantierbaren Impulsgenerators ist vorgesehen, dass der primäre Energiespeicher und die mehreren sekundären Energiespeicher in Reihe geschaltet sind, insbesondere fest oder unveränderlich, d.h. insbesondere ohne trennbare Schalter zwischen den Energiespeichern.

Gemäß einer alternativen Ausführungsform des implantierbaren Impulsgenerators ist vorgesehen, dass der primäre Energiespeicher und jeder der mehreren sekundären Energiespeicher in Reihe schaltbar sind, wobei insbesondere die sekundären Energiespeicher zueinander parallel schaltbar sind.

Insbesondere können dabei die mehreren sekundären Energiespeicher im Wesentlichen gleiche Kapazitäten (das heißt innerhalb einer Toleranz von nicht mehr als 20 %) und/oder Nennspannungen (das heißt innerhalb einer Toleranz von nicht mehr als 10 %) aufweisen oder unterschiedliche Kapazitäten und/oder Nennspannungen. Vorteilhafterweise handelt es sich bei mehreren sekundären Energiespeichern um jeweils um den gleichen oder identischen Typ.

Gemäß einer weiteren Ausführungsform des erfindungsgemäßen implantierbaren Impulsgenerators ist vorgesehen, dass im zweiten Intervall jeweils an einem Zeitpunkt jeweils einer der sekundären Energiespeicher (entweder abwechselnd oder hintereinander alle) über den therapeutischen Strompfad entladen wird. Auch hier wird der jeweilige Zeitpunkt vorzugsweise so gewählt, dass die Spannung des betreffenden Energiespeichers nicht unter einen vordefinierten Schwellenwert fällt, beispielsweise 80 % der Ausgangsspannung. Ebenso können die jeweiligen Zeitpunkte so gewählt werden, dass die Therapiespannung um nicht mehr als 20% der Maximalspannung bzw. Ausgangsspannung abfällt.

Vorzugsweise erfolgt die Entladung des oder der sekundären Energiespeicher in den therapeutischen Strompfad derartig, dass die Abweichung der tatsächlichen Impulsform (Therapiespitzenspannung/-strom) vom der gewünschten idealen rechteckigen Impulsform weniger als 50 %, vorzugsweise weniger als 20 % ist. Solche Abweichungen werden auch als *ripple* bezeichnet. Sichtbar ist dies als Zacke im Spannungsverlauf.

Die Abweichung (*ripple*) oder Abweichungen kann wie oben beschrieben durch eine Spannungsüberwachung in den genannten Grenzen gehalten werden, wobei die abfallende Spannung der exponentiellen Teilentladung des jeweiligen Energiespeichers beobachtet wird. Fällt die Spannung unter einen vordefinierten Schwellenwert, wird die Ladung eines anderen Energiespeichers abgerufen, wobei die Therapiespannung auf die gewünschte Maximalspannung steigt und mit der Entladung des anderen Energiespeichers wieder absinkt. Dies ist dann durch eine weitere Zacke (*ripple*) im Spannungsverlauf sichtbar.

Die Abweichung (*ripple*) oder Abweichungen können auch wie oben beschrieben auch zeitgesteuert kontrolliert werden. Vorzugsweise wird der letzte sekundäre Energiespeicher, dessen Ladung abgerufen wird, bis zu einer Umpolung bei einem biphasischen Schock entladen (der letzten Zacken in der Therapiespannung "klingt aus" bis Umpolung des Schocks).

Der Zeitpunkt der Umschaltung zum nächsten Energiespeichers kann beispielsweise aus dem Exponentialgesetz der Energiespeicherentladung bei bekannter Zeitkonstante, mit bekannter Kapazität C und dem wie oben beschrieben bestimmten Widerstand R so ermittelt werden, dass die Ladungsmenge des zugeschalteten Energiespeichers der ersten Polarität gleich ist (+/-20%).

Die Abweichung (*ripple*) oder Abweichungen von der idealen Impulsform können so kontrolliert werden, dass die Therapiespannung bei jedem Umschalten auf den nächsten Energiespeicher im Wesentlichen (+/- 20 %) auf die gleiche Spannung steigt, d. h. die Spitzen der *ripple* liegen auf gleicher Höhe (+/- 20).

Alternativ können die Abweichung oder die Abweichungen von der idealen Impulsform so kontrolliert werden, dass die Therapiespannung bei jedem Umschalten auf den nächsten Energiespeicher absinkt, beispielsweise jedes Mal um max. 20 %.

Die vorgenannte Kontrolle der Abweichung oder Abweichungen kann wo oben beschrieben zeitgesteuert oder spannungsgesteuert werden.

Gemäß einer weiteren Ausführungsform des erfindungsgemäßen implantierbaren Impulsgenerators ist vorgesehen, dass die Steuereinheit weiterhin dazu ausgebildet ist, in einer zweiten Phase der Impulsabgabe mindestens einen elektrischen Schalter, vorzugsweise mehrere Schalter einer Brückenschaltung (H-Brücke), der elektrischen Schaltung derartig anzusteuern, dass sich die Stromrichtung in dem therapeutischen Strompfad umkehrt. Dadurch kann vorteilhafterweise ein steiler Spannungsabfall des Impulses am Ende der ersten Phase erreicht und ein biphasischer therapeutischer Impuls erzeugt werden. Außerdem können vorteilhafterweise Bereiche im Zielgewebe in der zweite Phase stimuliert werden, die in der ersten Phase nicht ausreichend stimuliert wurden. Gleichzeitig kann damit vorteilhafterweise ein Ladungsausgleich im Zielgewebe, vorzugsweise dem Herzgewebe, erreicht werden.

Gemäß einer weiteren Ausführungsform des erfindungsgemäßen implantierbaren Impulsgenerators ist vorgesehen, dass die Steuereinheit weiterhin dazu ausgebildet ist, in einer zweiten Phase der Impulsabgabe:
- einen elektrischen Schalter in der elektrischen Schaltung derart anzusteuern, dass der primäre Energiespeicher über den therapeutischen Strompfad entladen wird, oder
- einen elektrischen Schalter in der elektrischen Schaltung derart anzusteuern, dass der primäre Energiespeicher und der oder die sekundären Energiespeicher über den therapeutischen Strompfad entladen werden.

Gemäß einer alternativen Ausführungsform des erfindungsgemäßen implantierbaren Impulsgenerators ist vorgesehen, dass die Steuereinheit weiterhin dazu ausgebildet ist, am Ende der ersten Phase der Impulsabgabe einen oder mehrere elektrische Schalter derartig anzusteuern, dass der primären Energiespeicher und/oder die der oder die mehreren sekundären Energiespeicher vom therapeutischen Strompfad getrennt werden bzw. in einem oder mehreren damit verbunden Ableitwiderständen entladen werden. Vorteilhafterweise kann auch mit dieser Ausführungsform ein steiler Spannungsabfall des Impulses am Ende der ersten Phase erreicht werden, wobei hierbei ein monophasischer Impuls erzeugt werden kann.

Gemäß einer weiteren Ausführungsform des erfindungsgemäßen implantierbaren Impulsgenerators ist vorgesehen, dass der primäre Energiespeicher aus einer Vielzahl von individuellen Energiespeichern besteht, die zueinander in Reihe oder parallel geschaltet sind. Vorzugsweise werden die Anzahl der individuellen Energiespeicher der benötigten oder gewünschten Ladungskapazität angepasst. Vorzugsweise umfasst der primäre Energiespeicher mindestens zwei individuelle Energiespeicher, bevorzugt drei individuelle Energiespeicher.

Gemäß einer weiteren Ausführungsform des erfindungsgemäßen implantierbaren Impulsgenerators ist vorgesehen, dass die individuellen Energiespeicher des primären Energiespeichers fest in Reihe geschaltet sind.

Insbesondere können die individuellen Energiespeicher des primären Energiespeichers im Wesentlichen gleiche Kapazitäten (das heißt innerhalb einer Toleranz von nicht mehr als 20%) und/oder Nennspannungen (das heißt innerhalb einer Toleranz von nicht mehr als 10%) aufweisen oder unterschiedliche Kapazitäten und/oder Nennspannungen.

Gemäß einer weiteren Ausführungsform des erfindungsgemäßen implantierbaren Impulsgenerators ist vorgesehen, dass der oder die Schalter der elektrischen Schaltung elektronischen Schalter bzw. Halbleiterschalter sind, insbesondere ausgewählt aus: Bipolartransistor mit isolierter Gate-Elektrode (*insulated-gate bipolar transistor,* IGBT), AGT (*anode gated thyristor*) oder eine Kombination der vorgenannten elektronischen Schalter.

Gemäß einer weiteren Ausführungsform des erfindungsgemäßen implantierbaren Impulsgenerators ist vorgesehen, dass mindestens einer der oben genannten Schalter in elektrischer Verbindung mit einer Diode steht, wobei die Diode zwischen einem der Energiespeicher und dem Schalter angeordnet ist. Die Diode ist vorzugsweise dazu ausgebildet, den Strom in Richtung des Energiespeichers zu sperren. Vorzugsweise ist zwischen jedem Schalter und dem dazu gehörigen Energiespeicher eine Diode angeordnet, ausgenommen den Schalter, mit welchem der letzte sekundäre Energiespeicher über den therapeutischen Strompfad entladen wird. Auch hier ist jede der Dioden vorzugsweise dazu ausgebildet, den Strom in Richtung des jeweiligen Energiespeichers zu sperren. Vorteilhafter Weise lassen sich damit die Schalter vor Verpolung schützen.

Gemäß einer weiteren Ausführungsform des erfindungsgemäßen implantierbaren Impulsgenerators ist vorgesehen, dass die mehreren sekundären Energiespeicher zwischen zwei und vier sekundäre Energiespeicher umfassen, vorzugsweise drei sekundäre Energiespeicher.

Gemäß einer Ausführungsform des erfindungsgemäßen implantierbaren Impulsgenerators ist vorgesehen, dass die Energiespeicher Kondensatoren oder Spulen sind. Hierbei können mehrere Energiespeicher, beispielsweise mehrere sekundäre Energiespeicher durch einen Kondensator gebildet werden, welcher mehrere Kapazitäten umfasst, beispielsweise die durch mehrere Anoden gebildet werden können, wobei die mehreren Kapazitäten unabhängig voneinander entladen werden können.

Dementsprechend ist gemäß einer weiteren Ausführungsform des erfindungsgemäßen implantierbaren Impulsgenerators vorgesehen, dass die mehreren sekundären Energiespeicher durch einen Kondensator mit mindestens einer ersten Elektrode mit einer ersten Polarität und mindestens zwei zweite Elektroden mit einer zweiten Polarität ist, wobei die erste Elektrode und die mindestens zwei zweiten Elektroden separat voneinander von der Außenseite des Kondensators elektrisch kontaktierbar sind.

Gemäß einer weiteren Ausführungsform des erfindungsgemäßen implantierbaren Impulsgenerators ist vorgesehen, dass der primäre Energiespeicher und/oder der oder die sekundären Energiespeicher ein Kondensator mit mindestens einer Kathode und mindestens zwei Anoden ist, vorzugsweise mit drei Anoden, wobei die Kathode und die mindestens zwei Anoden separat voneinander von der Außenseite des Kondensators elektrisch kontaktierbar sind. Vorzugsweise umfasst der Kondensator ein elektrisch leitfähiges Gehäuse, in welchem die Kathode und die Anoden angeordnet sind, wobei das Gehäuse in elektrischer Verbindung mit der Kathode steht, und die Anoden elektrisch vom Gehäuse und voneinander über mindestens eine elektrische Durchführung von der Außenseite des Kondensators bzw. des Kondensatorgehäuse elektrisch kontaktierbar sind. Vorzugsweise wird dabei die Kathode von einem Elektrolyten gebildet, wobei die Anoden vorzugsweise aus einem Ventilmetall, vorzugsweise Aluminium, Tantal oder Niob, gebildet werden. Insbesondere kann dabei jede der Anode jeweils eine eigene Kapazität mit der Kathode (dem Elektrolyten) bilden, welche vorzugsweise im Bereich von 200 µF bis 300 µF, insbesondere bei ca. 241 µF.

Gemäß einer weiteren Ausführungsform des erfindungsgemäßen implantierbaren Impulsgenerators ist vorgesehen, dass die elektrische Schaltung einen primären Energiespeicher umfasst, welcher drei der im vorherigen Absätze beschriebenen Kondensatoren mit Mehrfachelektroden umfasst, und einen sekundären Energiespeicher, der von einem in vorherigen Absatz beschriebenen Kondensator gebildet wird, wobei insbesondere der als sekundäre Energiespeicher ausgebildete Kondensator mindestens zwei, vorzugsweise drei Anoden umfasst, die unabhängig voneinander von der Außenseite des Kondensators elektrisch kontaktierbar sind und entsprechend mindestens zwei, vorzugsweise drei, unabhängig voneinander entladbare Kapazitäten bilden können.

Gemäß einer weiteren Ausführungsform des erfindungsgemäßen implantierbaren Impulsgenerators ist vorgesehen, dass der oder die Kondensatoren, welche den primären Energiespeicher, die individuellen Energiespeicher oder einen der vorgenannten sekundären Energiespeicher bilden, Elektrolytkondensatoren oder Keramikkondensatoren oder Filmkondensatoren sind, vorzugsweise Aluminium- oder Tantal-Elektrolytkondensatoren, vorzugsweise mit einer Energiedichte von mindestens 5 J^{∗}cm³. Gemäß einer Ausführungsform des erfindungsgemäßen Energiespeichers ist vorgesehen, dass
- der primäre Energiespeicher eine Kapazität im Bereich von 150 µF bis 300 µF und/oder eine Nennspannung im Bereich von 250 V bis 300 V hat, und/oder
- der oder die sekundären Energiespeicher unabhängig voneinander eine Kapazität im Bereich von 180 µF bis 36 µF und/oder eine Nennspannung im Bereich von 250 V bis 255 V haben.

Sofern der primäre Energiespeicher aus mehreren individuellen Energiespeichern gebildet wird, beispielsweise zwei oder drei, weist der primäre Energiespeicher vorzugsweise eine Gesamtkapazität im Bereich von 150 µF bis 300 µF auf.

Gemäß Anspruch 11 wird ein Verfahren zur Abgabe eines elektrischen Impulses mit einer im Wesentlichen rechteckigen Impulsform zur Verfügung gestellt. Das Verfahren umfasst die Schritte:
- Verbinden eines geladenen primären Energiespeichers mit einem Entladestrompfad in einem ersten Intervall einer ersten Phase einer Impulsabgabe; und
- Verbinden von mindestens einem geladenen sekundären Energiespeicher mit dem Entladestrompfad in einem zweiten Intervall der ersten Phase der Impulsabgabe.

Vorteilhafterweise ist das erfindungsgemäße Verfahren mit dem erfindungsgemäßen implantierbaren Impulsgenerator gemäß Anspruch 1 oder einer der oben aufgeführten Ausführungsformen durchführbar.

Gemäß einer Ausführungsform des erfindungsgemäßen Verfahrens ist vorgesehen, dass der geladene primäre Energiespeicher und der mindestens eine geladene sekundäre Energiespeicher fest in Reihe geschaltet sind, d.h. insbesondere nicht durch Schalter getrennt sind.

Gemäß einer alternativen Ausführungsform des erfindungsgemäßen Verfahrens ist vorgesehen, dass der geladene primäre Energiespeicher und der mindestens eine geladene sekundäre Energiespeicher in Reihe schaltbar sind.

Gemäß einer Ausführungsform des erfindungsgemäßen Verfahrens ist vorgesehen, dass das Verbinden des mindestens einen sekundären Energiespeichers mit dem Entladestrompfad signal- oder zeitgesteuert wird.

Gemäß einer Ausführungsform des erfindungsgemäßen Verfahrens ist vorgesehen, dass im zweiten Intervall der ersten Phase der Impulsabgabe mehrere geladene sekundäre Energiespeicher sequentiell oder hintereinander mit dem Entladestrompfad verbunden werden.

Gemäß einer Ausführungsform des erfindungsgemäßen Verfahrens ist vorgesehen, dass der geladene primäre Energiespeicher und die mehreren geladenen sekundären Energiespeicher fest in Reihe geschaltet sind, d.h. insbesondere nicht durch Schalter getrennt sind.

Gemäß einer alternativen Ausführungsform des erfindungsgemäßen Verfahrens ist vorgesehen, dass der geladene primäre Energiespeicher und die mehreren geladene sekundäre Energiespeicher in Reihe schaltbar sind.

Gemäß einer Ausführungsform des erfindungsgemäßen Verfahrens ist vorgesehen, dass immer jeweils nur ein geladener sekundäre Energiespeicher mit dem Entladestrompfad verbunden wird, wobei insbesondere jeder der mehreren geladenen sekundären Energiespeicher hintereinander mit dem Entladestrompfad verbunden werden. Dies kann insbesondere durch eine elektrische Schaltung erreicht werden, in welchem die sekundären Energiespeicher zueinander parallel schaltbar sind. Damit sind immer nur ein sekundärer Energiespeicher und der primäre Energiespeicher mit dem Entladestrompfad verbunden.

Gemäß einer Ausführungsform des erfindungsgemäßen Verfahrens ist vorgesehen, dass die mehreren geladenen sekundären Energiespeicher sequentiell mit dem Entladestrompfad verbunden werden, wobei insbesondere nacheinander alle sekundären Energiespeicher mit dem Entladestrompfad verbunden werden. Dies kann insbesondere durch eine elektrische Schaltung erreicht werden, in welcher der primäre Energiespeicher und die sekundären Energiespeicher zueinander in Reihe geschaltet sind. Damit wird der primäre Energiespeicher, ein erster sekundärer Energiespeicher und jeder weitere sekundäre Energiespeicher hintereinander in Reihe mit dem Entladestrompfad verbunden.

Gemäß einer weiteren Ausführungsform des erfindungsgemäßen Verfahrens ist vorgesehen, dass das Verbinden des primären Energiespeichers und des mindestens einen sekundären Energiespeichers oder der mehreren sekundären Energiespeicher jeweils mittels eines Schalters, insbesondere mittels eines elektronischen Schalters, ausgeführt wird.

Gemäß einer weiteren Ausführungsform des erfindungsgemäßen Verfahrens ist vorgesehen, dass in einer zweiten Phase der Impulsabgabe die Stromrichtung im Strompfad umgekehrt wird. Eine solche Umkehrung der Stromrichtung kann in zweckdienlicher Weise mittels einer Brückenschaltung realisiert werden.

Gemäß einer weiteren Ausführungsform des erfindungsgemäßen Verfahren ist vorgesehen, dass in der zweiten Phase der Impulsabgabe,
- nur der primäre Energiespeicher mit dem Entladestrompfadverbunden ist, oder
- der primäre Energiespeicher und der oder die sekundären Energiespeicher mit dem Entladestrompfad verbunden sind.

Weitere Merkmale und Vorteile der Erfindung werden nachfolgend anhand der Figurenbeschreibung von Ausführungsbeispielen erläutert. Es zeigen:
- Fig. 1: diverse Schockformen von implantierbaren Defibrillatoren und die Auswirkung auf die Transmembranspannung von Herzmuskelzellen.
- Fig. 2: (A) eine Ausführungsform einer erfindungsgemäßen Schaltung mit in Reihe schaltbaren primären und sekundären Energiespeichern, (B) den dazu gehörigen Spannungsverlauf der Therapiespannung und der Energiespeicher (oben) und dessen Auswirkung auf die Transmembranspannung der Herzmuskelzellen (unten), und (C) eine detailliertere Darstellung der in (a) gezeigten Ausführungsform.
- Fig. 3: (A) eine alternative Ausführungsform einer erfindungsgemäßen Schaltung mit ein Reihe schaltbaren primären Energiespeichern und parallel schaltbaren sekundären Energiespeichern, und (B) den dazu gehörigen Spannungsverlauf der Therapiespannung und der Energiespeicher (oben) und dessen Auswirkung auf die Transmembranspannung der Herzmuskelzellen (unten),
- Fig. 4: eine alternative Ausführungsform einer erfindungsgemäßen Schaltung unter Verwendung von Kondensatoren mit von außen kontaktierbaren Mehrfachanoden.

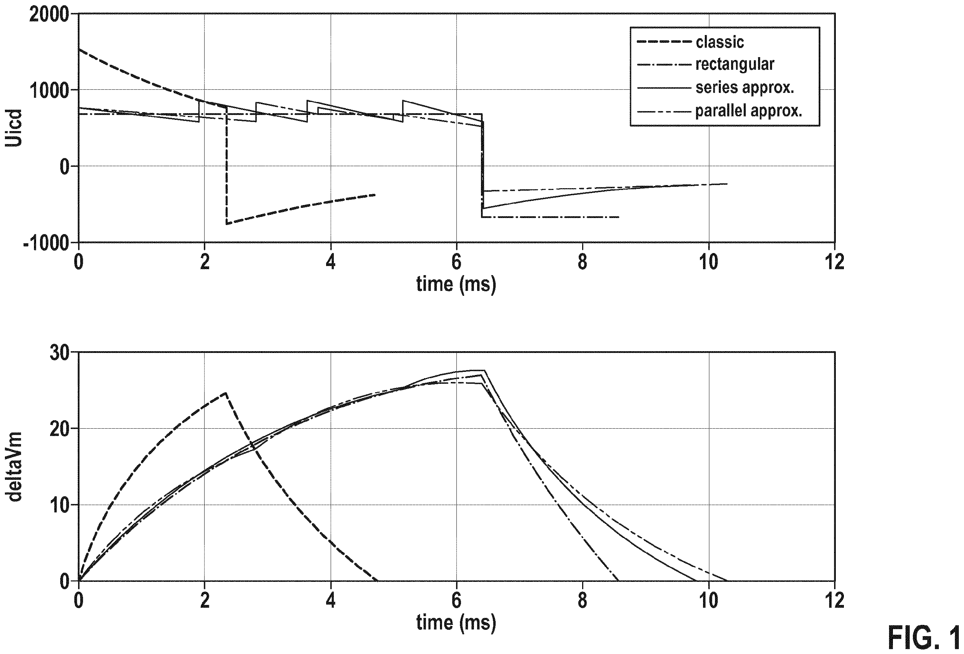

Figur 1 zeigt den Stand der Technik (obere Graphik, blau) der Schockform eines implantierbaren Defibrillators (zutreffend sowohl für transvenöse als auch subkutane ICDs). Die Therapiespannung erfolgt aus der Entladung effektiv nur eines Kondensators und ist daher exponentiell abfallend. Dies hat den Nachteil, dass bei wesentlich höheren Startspannungen begonnen werden muss, um den gleichen Effekt im Herzen zu erzeugen. Vergleichsweise ist der ideale Verlauf eines Shocks mit rechteckiger erster Phase dargestellt (rot) sowie die Schockformen der erfindungsgemäßen Lösung, die den rechteckigen Verlauf approximieren.

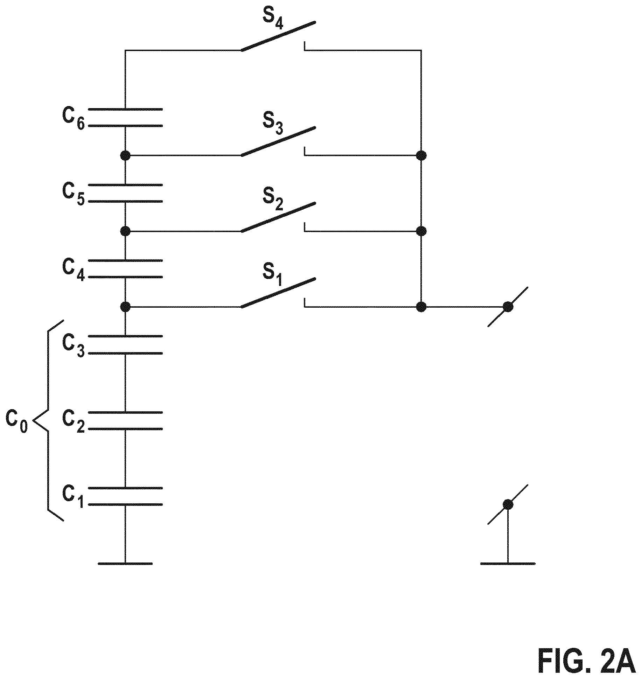

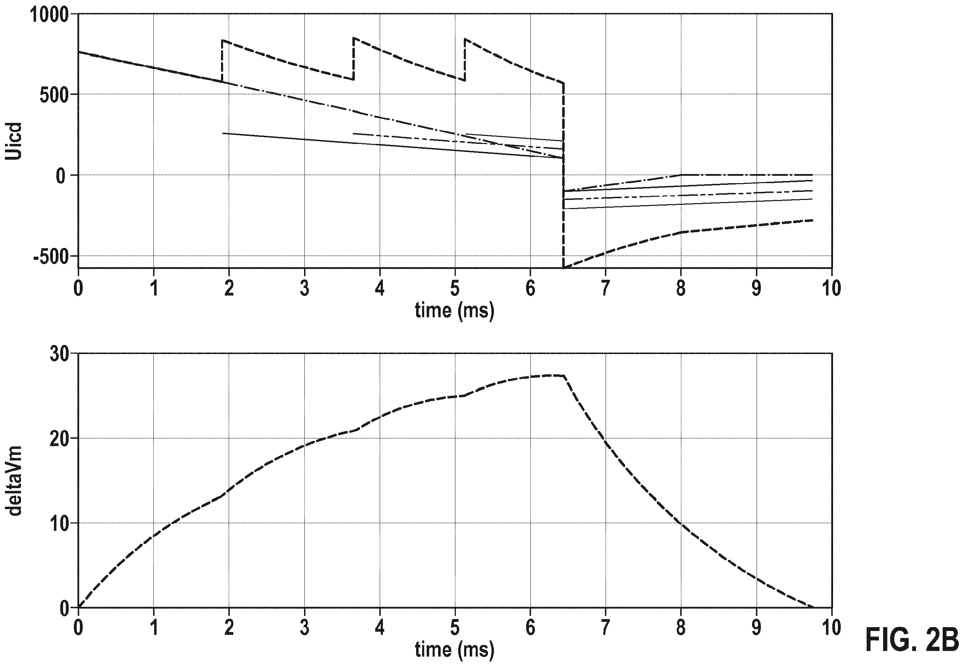

Figur 2A zeigt eine bevorzugte Ausführungsform der erfindungsgemäßen Schaltung, in der die Energiespeicher C₁ bis C₆ Kondensatoren sind, die in Reihe geschaltet bzw. schaltbar sind. Die Kondensatoren C₁ bis C₃, welchen den primären Energiespeicher gemäß der vorliegenden Erfindung bilden, können auch als eine Kapazität C₀ realisiert werden. Alle Kondensatoren C₁ bis C₆ können quasi gleichzeitig durch eine Ladeschaltung geladen werden. Die Schalter S₁-S₄ werden zur Therapieabgabe in aufsteigender Reihenfolge nacheinander geschaltet. Dabei wird Schalter i wieder geöffnet, bevor Schalter i+1 geschlossen wird. Diese Schaltung speist ferner die H-Brücke zur Erzeugung der zweiten Phase (nicht dargestellt). In der zweiten Phase wird einer der Schalter geschlossen, bevorzugt S₄. An Stelle von Schalter S4 kann auch eine Diode angeordnet sein, die den Strom in Richtung C₀/C₄ sperrt. Figur 2B zeigt in der oberen Abbildung den mit dieser elektrischen Schaltung erzielbaren Spannungsverlauf der Therapiespannung (blau) sowie an den Kondensatoren C₀ (rot) C₄ (gelb) C₅ (lila) C₆ (grün) und in der unteren Abbildung die entsprechende Auswirkung auf die Transmembranspannung der Herzmuskelzellen. Hierbei weist der primäre Energiespeicher C₀, der aus drei individuellen Energiespeichern C₁ bis C₃ gebildet wird, vorzugsweise eine Gesamtkapazität im Bereich von 150 µF bis 300 µF auf, und jeder der sekundären Energiespeicher C₄ bis C₆ vorzugsweise eine Kapazität im Bereich von 180 µF bis 360 µF.

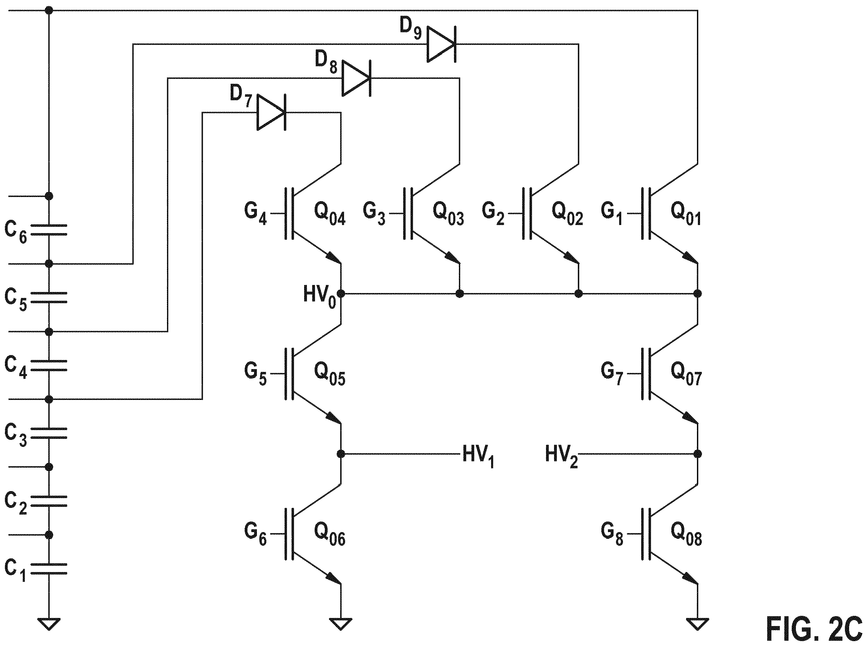

Fig. 2C zeigte eine Implementierung der in Fig. 2A dargestellten Ausführungsform mit elektronischen Schaltern und bis zu 6 Kondensatoren als Energiespeicher C₁-C₆ des erfindungsgemäßen Impulsgenerators. Davon sichern 3 Kondensatoren C₁, C₂, C₃ die ausreichend hohe Startspannung, die weiteren 3 Kondensatoren C₄, C₅, C₆ erzeugen den erwünschten annährend rechteckigen Impulsverlauf (Sägezahnverlauf), der in diesem Fall bis zu 4 Zacken aufweisen kann. Um den Schock biphasisch zu gestalten, wird herkömmlich eine H-Schaltung bestehend aus den elektronischen Schaltern (IGBTs) Q05-Q08 eingesetzt. Diese wird über die elektronischen Schalter (IGBTs) Q01-Q04 gespeist, die die Kondensatoren C₄, C₅, C₆ zuschalten. Die IGBTs Q02-Q04 werden vorzugsweise über die Dioden D7-D9 vor Verpolung geschützt. Der Schock wird über die Anschlüsse HV1-HV2 in den Körper geleitet. Geladen werden die Kondensatoren über eine Hochspannungsquelle die an HVin und Masse angeschlossen wird.

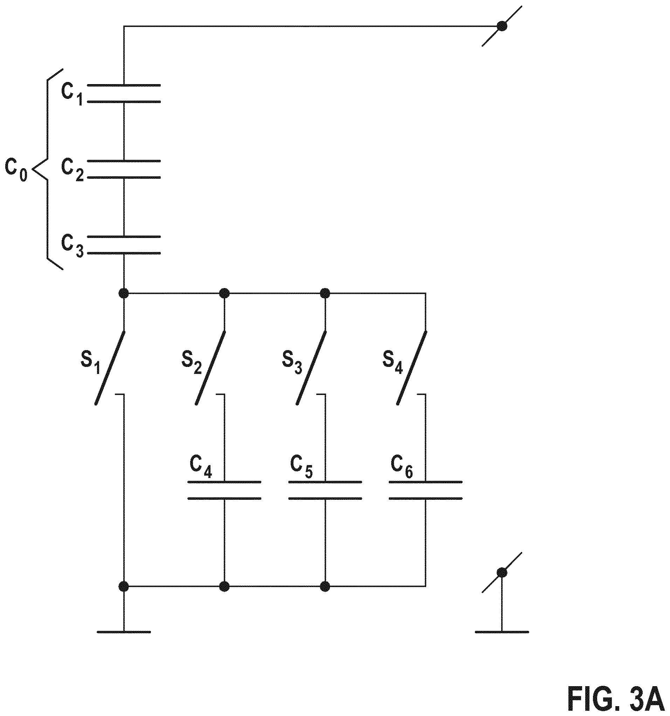

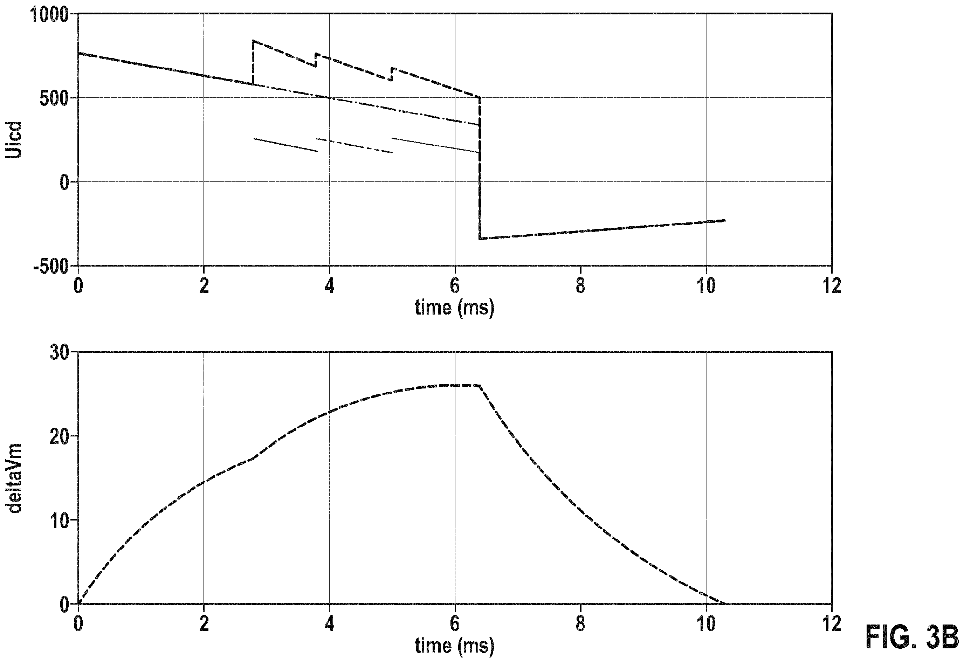

Figur 3A zeigt eine weitere bevorzugte Ausführungsform der erfindungsgemäßen elektrischen Schaltung mit Kondensatoren C₁ bis C₆ als Energiespeicher, wobei die hinzugeschalteten bzw. hinzuschaltbaren Energiespeicher C₄ bis C₆ parallel zueinander geschaltet bzw. schaltbar sind. Auch hier können die Kondensatoren C₁-C₃ können als eine Kapazität C₀ realisiert werden. Alle Kondensatoren werden quasi gleichzeitig durch eine Ladeschaltung geladen. Die Schalter S₁-S₄ werden zur Therapieabgabe in aufsteigender Reihenfolge nacheinander geschaltet. Dabei wird Schalter i wieder geöffnet bevor Schalter i+1 geschlossen wird. Diese Schaltung speist ferner die H-Brücke zur Erzeugung der zweiten Phase. In der zweiten Phase wird einer der Schalter geschlossen, bevorzugt S₁. Figur 3B zeigt in der oberen Abbildung den mit elektrischen Schaltung erzielbaren Spannungsverlauf der Therapiespannung (blau) sowie an den Kondensatoren C₀ (rot) C₄ (gelb) C₅ (lila) C₆ (grün) und in der unteren Abbildung die entsprechende Auswirkung auf die Transmembranspannung der Herzmuskelzellen.

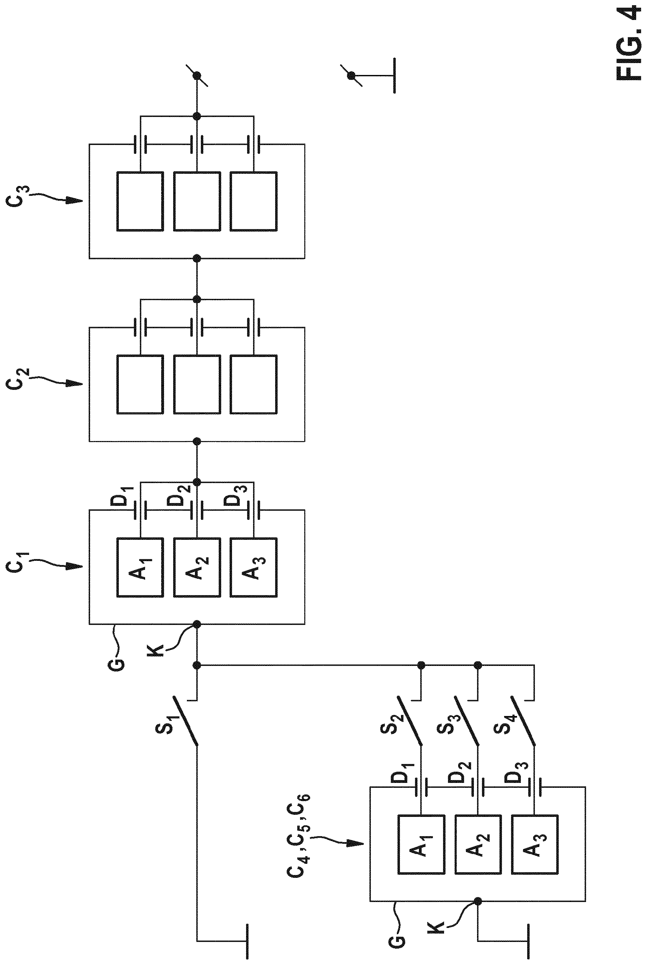

Figur 4 zeigt eine bevorzugte Ausführungsform der erfindungsgemäßen elektrischen Schaltung mit einem parallelen Ansatzes unter Verwendung von Kondensatoren mit Mehrfachanoden K, A₁, A₂, A₃ als primärer Energiespeicher C₀, C₁, C₂, C₃ und mehrere sekundäre Energiespeicher C₄ bis C₆. Insbesondere werden hierbei Kondensatoren verwendet, welche jeweils eine Kathode K und, beispielsweise, drei Anoden A₁, A₂, A₃ aufweisen, wobei die Kathode K vorteilhafterweise durch ein elektrisch leitfähiges Gehäuse von außen kontaktierbar ist, und die Anoden separat und elektrisch isoliert voneinander und vom Gehäuse G, beispielsweise über Durchführungen D₁, D₂, D₃, von außen kontaktierbar sind. Die erfindungsgemäßen mehreren sekundären Energiespeicher werden daher als ein Kondensator realisiert, der mit seinen drei separat kontakttierbaren Anoden A₁, A₂, A₃, drei separat voneinander entladbare Kapazitäten zur Verfügung stellt.

Denkbar wären jedoch auch, dass die Kondensatoren C₁ bis C₃, welchen den primären Energiespeicher gemäß der vorliegenden Erfindung bilden, derartig ausgeführt sind, dass die Anoden A₁, A₂, A₃ im Inneren des Gehäuses G elektrisch verbunden sind und über einen gemeinsamen Anodendraht, der beispielsweise über eine Durchführung nach außen geführt wird, von außen elektrisch kontaktierbar sind.

## Patentansprüche

1. Implantierbarer Pulsgenerator mit einer elektrischen Schaltung, wobei die elektrisch Schaltung umfasst:
- einen primären Energiespeicher (C₀, C₁, C₂, C₃),
- mindestens einen sekundäre Energiespeicher (C₄, C₅, C₆), und
- eine Steuereinheit, wobei die Steuereinheit dazu ausgebildet ist,
• einen elektrischen Schalter (S₁, Q04) in der elektrischen Schaltung derart anzusteuern, dass in einem ersten Intervall einer ersten Phase einer Impulsabgabe der primäre Energiespeicher (C₀, C₁, C₂, C₃) über einen therapeutischen Strompfad entladen wird, und
• einen elektrischen Schalter (S₂, S₃, S₄, Q03, Q02, Q01) in der elektrischen Schaltung derart anzusteuern, dass in einem zweiten Intervall der ersten Phase der Impulsabgabe der primäre Energiespeicher (C₀, C₁, C₂, C₃) und der mindestens eine sekundäre Energiespeicher (C₄, C₅, C₆) über den therapeutischen Strompfad entladen wird,
wobei
der primäre Energiespeicher (C₀, C₁, C₂, C₃) und der mindestens eine sekundäre Energiespeicher (C₄, C₅, C₆) fest in Reihe geschaltet sind oder schaltbar sind.

2. Der Implantierbare Pulsgenerator nach Anspruch 1, **dadurch gekennzeichnet, dass** die elektrische Schaltung mehrere sekundäre Energiespeicher (C₄, C₅, C₆) aufweist, wobei der primäre Energiespeicher (C₀, C₁, C₂, C₃) und die mehreren Energiespeicher (C₄, C₅, C₆) fest in Reihe geschaltet sind oder schaltbar sind, und wobei die Steuereinheit dazu ausgebildet ist, einen oder mehrere elektrische Schalter (S₂, S₃, S₄) der elektrischen Schaltung derart anzusteuern, dass im zweiten Intervall der ersten Phase der Impulsabgabe die sekundären Energiespeicher (C₄, C₅, C₆) sequentiell oder hintereinander über den therapeutischen Strompfad entladen wird.

3. Der Implantierbare Pulsgenerator nach Anspruch 1 oder 2, **dadurch gekennzeichnet, dass** die Steuereinheit weiterhin dazu ausgebildet ist, in einer zweiten Phase der Impulsabgabe, mindestens einen elektrischen Schalter, vorzugsweise mehrere Schalter (Q05, Q06, Q07, Q08) einer Brückenschaltung, derartig anzusteuern, dass sich die Stromrichtung in dem therapeutischen Strompfad umkehrt.

4. Der implantierbare Pulsgenerator nach einem der vorherigen Ansprüche, **dadurch gekennzeichnet, dass** die Steuereinrichtung weiterhin dazu ausgebildet ist, einen elektrischen Schalter (S₂, S₃, S₄) in der elektrischen Schaltung derart anzusteuern, dass im zweiten Intervall
- der primäre Energiespeicher (C₀, C₁, C₂, C₃) und hintereinander alle sekundären Energiespeicher (C₄, C₅, C₆) über den therapeutischen Strompfad entladen werden, oder
- der primäre Energiespeicher (C₀, C₁, C₂, C₃) und jeweils einer der mehreren sekundären Energiespeicher (C₄, C₅, C₆) über den therapeutischen Strompfad entladen werden.

5. Der implantierbare Pulsgenerator nach einem der vorherigen Ansprüche, **dadurch gekennzeichnet, dass** der primäre Energiespeicher (Co) aus einer Vielzahl von individuellen Energiespeichern (C₁, C₂, C₃) besteht, die fest in Reihe geschaltet sind oder zueinander parallel schaltbar sind.

6. Der implantierbare Pulsgenerator nach einem der vorherigen Ansprüche, **dadurch gekennzeichnet, dass** der oder die Schalter (S₁, S₂, S₃, S₄, Q01, Q02, Q03, Q04, Q05, Q06, Q07, Q08) der elektrische Schaltung elektronische Schalter sind, insbesondere ausgewählt aus: IGBT, AGT oder eine Kombination der vorgenannten elektronischen Schalter.

7. Der implantierbare Pulsgenerator nach einem der vorherigen Ansprüche, **dadurch gekennzeichnet, dass** die elektrische Schaltung zwischen zwei und vier sekundäre Energiespeicher umfassen, vorzugsweise drei sekundäre Energiespeicher (C₄, C₅, C₆).

8. Der implantierbare Pulsgenerator nach einem der vorherigen Ansprüche, **dadurch gekennzeichnet, dass** der primäre Energiespeicher (C₀, C₁, C₂, C₃) und/oder der oder die sekundären Energiespeicher (C₄, C₅, C₆) unabhängig voneinander ein Kondensator oder eine Spule sind.

9. Der implantierbare Pulsgenerator nach Anspruch 8, **dadurch gekennzeichnet, dass** die mehreren sekundären Energiespeicher (C₄, C₅, C₆) durch einen Kondensator mit mindestens einer Elektrode (K) mit einer ersten Polarität und mindestens zwei zweite Elektroden einer zweiten Polarität (A₁, A₂, A₃) gebildet werden, wobei jeder Elektroden (K, A₁, A₂, A₃) separat voneinander von der Außenseite des Kondensators elektrisch kontaktierbar sind.

10. Der implantierbare Pulsgenerator nach einem der vorherigen Ansprüche, **dadurch gekennzeichnet, dass**
- der primäre Energiespeicher (C₀) eine Kapazität im Bereich von 150 µF bis 300 µF und/oder eine Nennspannung im Bereich von 250 V bis 255 V hat, und/oder
- der oder die sekundären Energiespeicher (C₄, C₅, C₆) unabhängig voneinander eine Kapazität im Bereich von 180 µF bis 360 µF und/oder eine Nennspannung im Bereich von 250 V bis 255 V haben.

11. Verfahren zur Abgabe eines elektrischen Impulses mit einem im Wesentlichen rechteckigen Spannungsverlauf (Impulsform) umfassend die Schritte:
- Verbinden eines geladenen primären Energiespeichers (C₀, C₁, C₂, C₃) mit einem Entladestrompfad in einem ersten Intervall einer ersten Phase einer Impulsabgabe; und
- Verbinden mindestens eines sekundären Energiespeichers (C₄, C₅, C₆) mit dem Entladestrompfad in einem zweiten Intervall der ersten Phase der Impulsabgabe,
wobei der primäre Energiespeicher (C₀, C₁, C₂, C₃) und der mindestens eine sekundäre Energiespeicher (C₄, C₅, C₆) fest in Reihe geschaltet sind oder schaltbar sind.

12. Verfahren nach Anspruch 11, **dadurch gekennzeichnet, dass** im zweiten Intervall der ersten Phase der Impulsabgabe mehrere sekundäre Energiespeicher (C₄, C₅, C₆) mit dem Entladestrompfad verbunden werden.

13. Verfahren nach Anspruch 12, **dadurch gekennzeichnet, dass**:
- immer jeweils nur ein sekundäre Energiespeicher (C₄, C₅, C₆) mit dem Entladestrompfad verbunden wird, wobei insbesondere jeder der mehreren sekundären Energiespeicher (C₄, C₅, C₆) hintereinander mit dem Entladestrompfad verbunden wird, oder
- die mehreren sekundären Energiespeicher (C₄, C₅, C₆) sequentiell mit dem Entladestrompfad verbunden werden, wobei insbesondere nacheinander alle sekundären Energiespeicher (C₄, C₅, C₆) mit dem Entladestrompfad verbunden werden.

14. Verfahren nach einem der Ansprüche 11 bis 13, **dadurch gekennzeichnet, dass** das Verbinden des primären Energiespeichers (C₀, C₁, C₂, C₃) und des mindestens einen sekundären Energiespeichers (C₄, C₅, C₆) oder der mehreren sekundären Energiespeicher (C₄, C₅, C₆) jeweils mittels eines Schalters (S₁, S₂, S₃, S₄, Q01, Q02, Q03, Q04), insbesondere mittels eines elektronischen Schalters, ausgeführt wird.

15. Verfahren nach einem der Ansprüche 11 bis 14, **dadurch gekennzeichnet, dass** in einer zweiten Phase der Impulsabgabe die Stromrichtung im therapeutischen Strompfad umgekehrt wird.
